# EUROPEAN PATENT APPLICATION

(11) **EP 1 808 139 A1**
(43) Date of publication of application: **18.07.2007**
(21) Application number: 05805435.4
(22) Date of filing: 31.10.2005
(51) Int. Cl.: A61B 17/34, A61B 18/00

(54) **ULTRASONIC TROCAR AND METHOD OF USING ULTRASONIC TROCAR**

(30) Priority: 05.11.2004 JP 2004322612
(71) Applicant: Olympus Corporation, Tokyo 151-0072 (JP)
(72) Inventor: OSHIDA, Masami, . (JP)
(74) Representative: von Hellfeld, Axel
(86) International application number: PCT/JP2005/020038
(87) International publication number: WO 2006/049138

(57) **Abstract**

An ultrasonic trocar (10) includes a handpiece unit (20), a dilator (40), and a tubular cannula (70). The handpiece unit includes an ultrasonic transducer (22) and a probe (24). The handpiece unit forms a puncture hole in a somatic wall by a tip end of the probe with ultrasonic vibration transmitted to the probe tip end from the transducer. The dilator (40) includes a tubular body portion (44) and a sheath (42). The body portion (44) has a dilating portion (52) which expands the puncture hole of the body wall, and the probe is inserted through from a proximal end portion of the body portion. The sheath (42) is arranged in a state of protruding from a distal end portion of the body portion, and covers the peripheral surface of the probe with the probe tip end remained. The cannula (70) includes the dilator (40) inserted therethrough and is kept retained to the puncture hole expanded by the expanding portion.

## Description

### Technical Field

The present invention relates to an ultrasonic trocar which punctures a probe into a body cavity by using ultrasonic waves and expands the puncture hole, and retains a cannula used as a guide tube of an insertion instrument into the body cavity to a somatic wall of a patient.

### Background Art

For example, in Jpn. Pat. Appln. KOKAI Publication Nos. 2003-10197 and 2004-194731, an ultrasonic trocar having four members is disclosed. That is, the ultrasonic trocar includes a handpiece unit, a sheath, a dilator, and a cannula. On the outside of the handpiece unit, the sheath is removably arranged. On the outside of the sheath, the dilator is removably arranged. On the outside of the dilator, the cannula is arranged removably.

The handpiece unit has a probe that can transmit ultrasonic vibration and that forms a puncture hole in a somatic wall. The cannula is retained to the puncture hole on the somatic wall formed by the probe. The sheath covers a peripheral surface of the probe of the handpiece unit. The dilator expands the puncture hole formed by the probe to a diameter to which the cannula can be retained.

Consequently, in the case where the cannula is retained to the somatic wall, ultrasonic vibration is applied to the probe of the handpiece unit in a state in which these four members of handpiece unit, dilator, sheath, and cannula are assembled as described above, and the tip end of the probe is punctured into the somatic wall. Thereafter, the handpiece unit, i.e., the probe is removed with respect to the other three members, and the puncture hole is expanded by making the best of the expanding portion of the dilator. In this way, the puncture hole is expanded to the dilator outside diameter, and the cannula is retained onto the somatic wall with the inner peripheral surface adhering to the maximum outside diameter position of the dilator expanding portion. Thereafter, the sheath and the dilator are removed successively from the cannula, and the cannula is finally retained on the somatic wall.

In Jpn. Pat. Appln. KOKAI Publication Nos. 2003-10197 and 2004-194731, the ultrasonic trocar includes four components. When the ultrasonic trocar is applied, there's need to the four components assembled. Thus, the assembling work of the four components is annoying. When the cannula is retained into the body cavity, another three members are removed from the cannula, and removing work is annoying.

### Disclosure of Invention

The present invention is made to solve the above problems, and has as its object to provide an ultrasonic trocar to be able to be easily carried out the assembling work and removing work.

An ultrasonic trocar according to an aspect of the present invention includes a handpiece unit, a dilator, and a tubular cannula. The handpiece unit includes an ultrasonic transducer and a probe. The handpiece unit forms a puncture hole in a somatic wall by a tip end of the probe with ultrasonic vibration transmitted to the probe tip end from the transducer. The dilator includes a tubular body and a sheath. The body portion has a dilating portion which expands the puncture hole of the body wall, and the probe is inserted through from a proximal end portion of the body portion. The sheath is arranged in a state of protruding from a distal end portion of the body portion, and covers the peripheral surface of the probe with the probe tip end remained. The cannula includes the dilator inserted therethrough and is kept retained to the puncture hole expanded by the expanding portion.

### Brief Description of Drawings

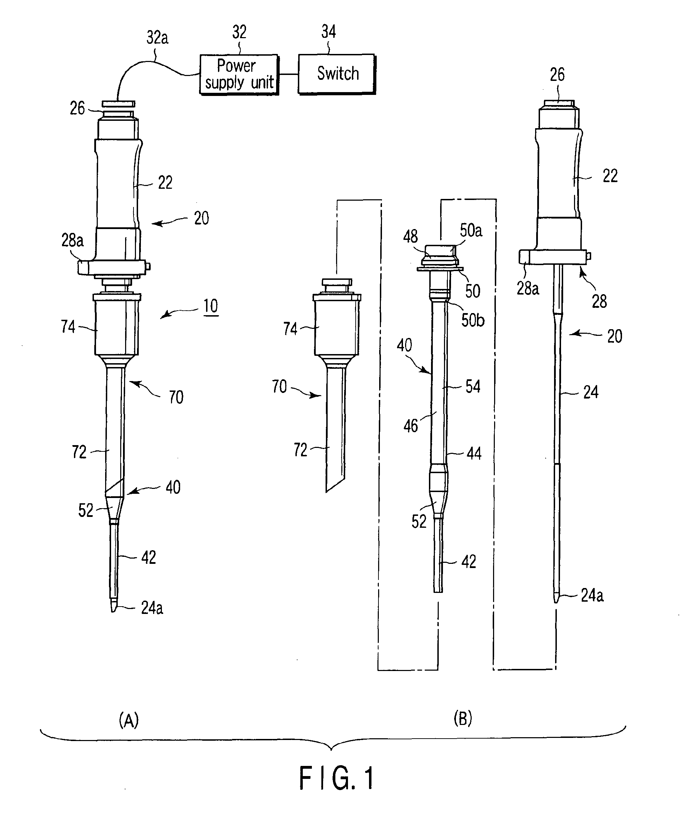

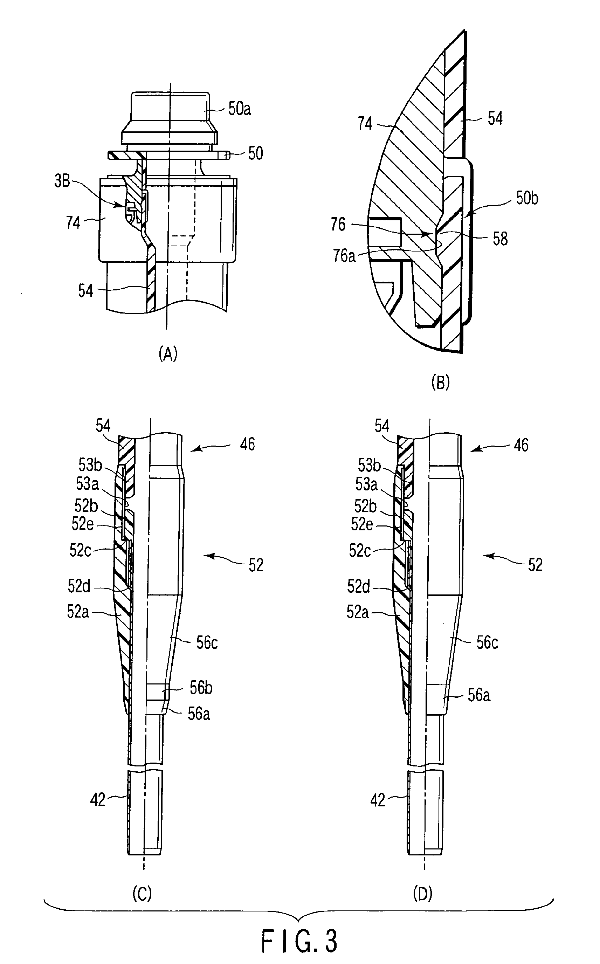

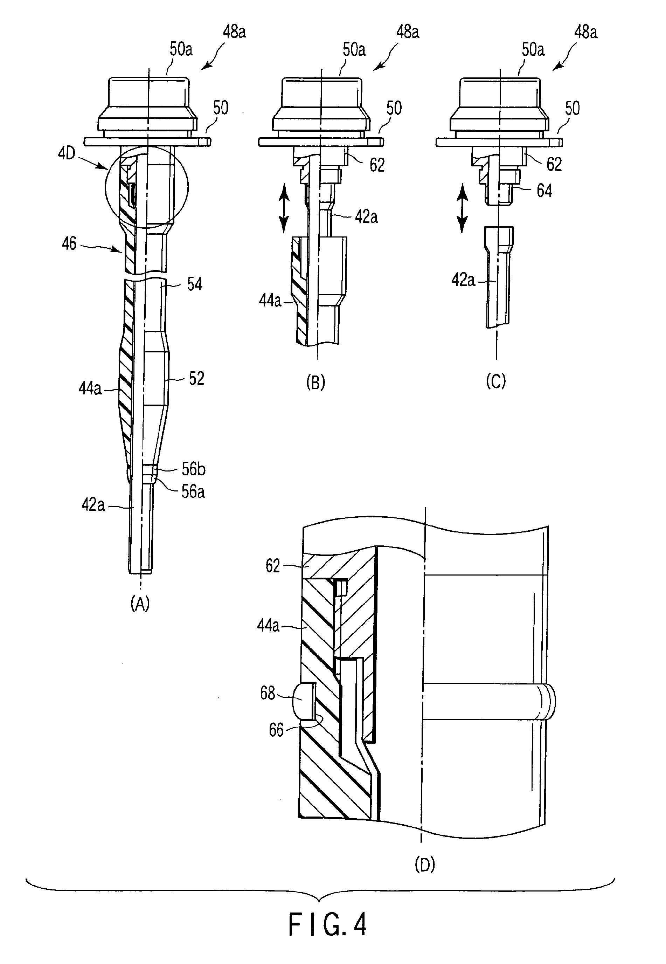

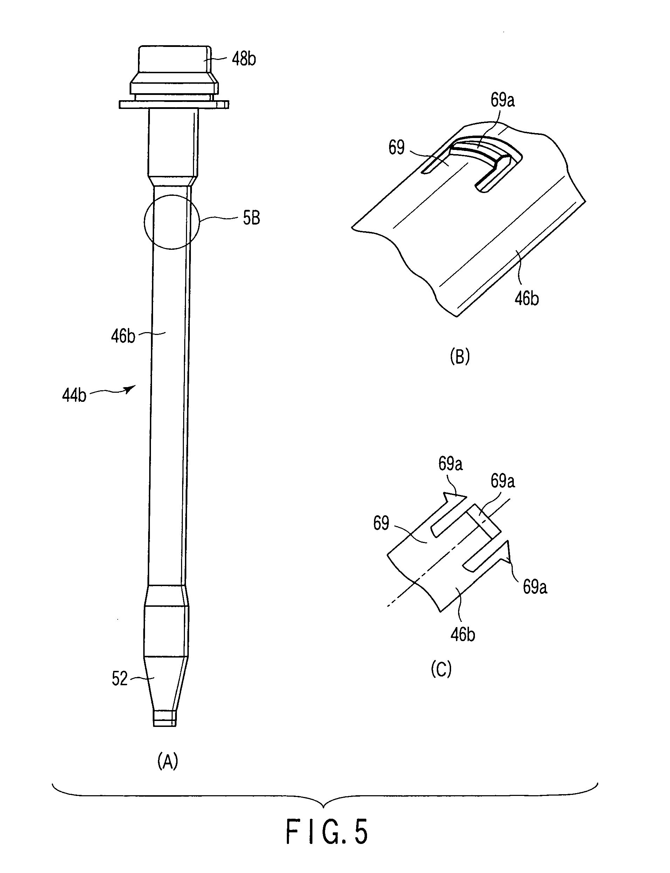

FIG. 1 shows an ultrasonic trocar according to a first embodiment, wherein (A) is a schematic view showing a state in which a handpiece unit, a dilator and a cannula are assembled; and (B) is a schematic view showing a state in which the handpiece unit, dilator, and cannula are separated from one another.

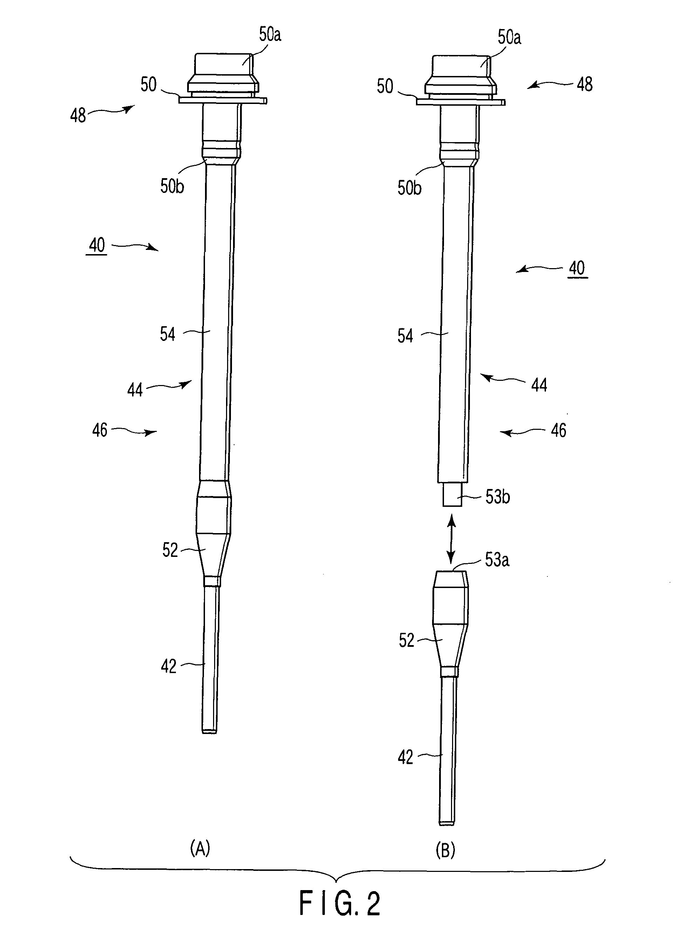

FIG. 2 shows a dilator in the ultrasonic trocar according to the first embodiment, wherein (A) is a schematic view showing a state in which an expanding portion of an insertion portion and a cylindrical portion of the insertion portion are assembled; and (B) is a schematic view showing a state in which the expanding portion and the cylindrical portion are separated from each other.
(A) of FIG. 3 is a schematic partial sectional view showing a holding portion of the dilator and the cannula in an assembled state of both in the ultrasonic trocar according to the first embodiment; (B) of FIG. 3 is a schematic partial sectional view showing an engaged state of the dilator and the cannula with the portion shown by symbol 3B in (A) of FIG. 3 enlarged; (C) of FIG. 3 is a schematic partial sectional view showing the expanding portion and a sheath in the ultrasonic trocar according to the first embodiment; and (D) of FIG. 3 is a schematic partial sectional view showing a deformed state of the expanding portion and sheath in the ultrasonic trocar according to the first embodiment with respect to (C) of FIG. 3.
(A) of FIG. 4 is a schematic partial sectional view showing a dilator in an ultrasonic trocar according to a second embodiment; (B) of FIG. 4 is a schematic partial sectional view showing a proximal end portion of the dilator in the ultrasonic trocar according to the second embodiment, and showing a state in which the body portion is attached and detached with a sheath mounted to a holding portion; (C) of FIG. 4 is a schematic partial sectional view showing the proximal end portion of the dilator in the ultrasonic trocar according to the second embodiment, and showing the state in which the sheath is attached to and detached from the holding portion; and (D) of FIG. 4 is an enlarged schematic partial sectional view showing the portion denoted by symbol 4D in (A) of FIG. 4.
(A) of FIG. 5 is a schematic view showing a body portion of a dilator in an ultrasonic trocar according to a third embodiment; (B) of FIG. 5 is an enlarged schematic view showing the portion denoted by symbol 5B in (A) of FIG. 5; and (C) of FIG. 5 is a modified example of an engaged portion shown in (B) of FIG. 5.

### Best Mode for Carrying Out the Invention

Hereinafter, best modes for carrying out the present invention will be described in detail with reference to the drawings.

First of all, a first embodiment will be described with reference to FIGS. 1 to 3.

(A) and (B) of FIG. 1 each show a configuration of an ultrasonic trocar 10 according to this embodiment.

As shown in (A) and (B) of FIG. 1, the ultrasonic trocar 10 according to this embodiment includes a handpiece unit 20, a dilator 40, and a cannula 70.

The handpiece unit 20 shown in (B) of FIG. 1 concentrically includes an ultrasonic transducer 22 and a probe (trocar inner needle) 24. The transducer 22 has an ultrasonic vibrator (not shown) and a substantially conical horn (not shown) which expands ultrasonic vibration emitted by the ultrasonic vibrator. At a proximal end portion (top end portion) of the transducer 22, a connector portion 26 for electrical connection is arranged.

As shown in (A) of FIG. 1, the other end of a cable 32a having one end connected to a power supply unit 32 is removably connected to the connector portion 26. A switch 34 which outputs signals to operate the power supply unit 32, such as for example, a foot switch and hand switch, is connected to the power supply unit 32. When predetermined signals are input to the power supply unit 32 from the switch 34 by operating the switch 34, the power supply unit 32 supplies electric power to the ultrasonic vibrator to generate ultrasonic vibration.

As shown in (B) of FIG. 1, to a distal end portion (bottom end portion of the transducer 22) of the horn, a proximal end portion (top end portion) of a long probe 24 as a straight needle-like member is removably connected by, for example, screws, etc. The probe 24 can transmit ultrasonic vibration and is punctured from a tip end portion (bottom end portion) 24a to a somatic wall. Consequently, the handpiece unit 20 can transmit to the tip end portion 24a of the probe 24 by further expanding the ultrasonic vibration which is generated by the ultrasonic vibrator of the ultrasonic transducer 22 and transmitted to the horn to be expanded.

A lock mechanism portion 28 is formed at the distal end portion of the transducer 22 outside of the proximal end portion of the probe 24. The lock mechanism portion 28 removably engages with a first attaching-and-detaching portion 50a (to be described later) of the proximal end portion (top end portion) of the dilator 40. The lock mechanism portion 28 has a disk-shaped concave portion (not shown) and an engaging pin (dilator loading and unloading button) 28a. This concave portion is formed at the distal end portion of the transducer 22 and around a longitudinal axis of the proximal end portion of the probe 24. The engaging pin 28a extends in a direction orthogonal to the axial direction of the probe 24. That is, the engaging pin 28a extends toward the central axis of the probe 24. The engaging pin 28a is energized by, for example, a spring in a direction orthogonal to the axial direction of the probe 24 and in a direction departing from the probe 24. The engaging pin 28a is engaged with the first attaching-and-detaching portion 50a of the proximal end portion of the dilator 40 when an operator presses the engaging pin 28a in a direction approaching the probe 24 to rotate.

Next, the dilator 40 will be explained.

The dilator 40 shown in (A) of FIG. 2 concentrically has a sheath 42 formed in a tubular shape and a body portion 44 formed in a tubular shape in the same manner. The sheath 42 provides heat resistance and slidability, and is formed with a flexible material. The sheath 42 is preferably formed with, for example, PTFE.

The body portion 44 includes an insertion portion 46 and a holding portion 48 provided to the proximal end portion (top end portion) of the insertion portion 46. The insertion portion 46 is inserted into the cannula 70, and the probe 24 of the handpiece unit 20 is inserted through the insertion portion 46. The body portion 44 may be formed with, for example, stainless steel but preferably, formed with a resin material such as polyphenyl sulfone.

The holding portion 48 includes a flange portion 50, and cylindrical first and second attaching-and-detaching portions 50a and 50b. The flange portion 50 is extended outwards in the radial direction in a flange shape. The first attaching-and-detaching portion 50a is provided above the flange portion 50. The second attaching-and-detaching portion 50b is provided below the flange portion 50.

The first attaching-and-detaching portion 50a can be attached to and detached from the concave portion of the lock mechanism portion 28 of the handpiece unit 20. A check valve (not shown) that prevents gas from the distal end portion side of the insertion portion 46 of the body portion 44 from passing through the holding portion 48 is removably mounted on the first attaching-and-detaching portion 50a. For example, a slit valve is used as the check valve.

As shown in (A) and (B) of FIG. 3, the second attaching-and-detaching portion 50b is elastically deformable, and has a protrusion 58 that protrudes outwards in the radial direction. The protrusion 58 of the second attaching-and-detaching portion 50b can be attached to and detached from a concave portion 76a of an engaging portion 76 (to be described later) of the cannula 70.

As shown in (A) and (B) of FIG. 2, the insertion portion 46 removably has a hard expanding portion 52 and a hard cylindrical portion 54 provided to a proximal end portion of the expanding portion 52. The expanding portion 52 expands a puncture hole formed by the tip end 24a (see FIG. 1) of the probe 24. The outside diameter of the cylindrical portion 54 is formed to be smaller than the maximum outside diameter of the expanding portion 52, and is formed so as to prevent friction from being generated with the inner circumferential surface of an insertion portion 72 of the cannula 70.

As shown in (B) of FIG. 2, fitting portions 53a and 53b capable of being fitted to each other are formed to the proximal end portion of the expanding portion 52 and the distal end portion of the hard cylindrical portion 54. These fitting portions 53a and 53b are preferably, for example, male screws or female screws, or click mechanisms. In this case, the explanation is made by assuming that a male screw portion is formed to the fitted portion 53a at the proximal end portion of the expanding portion 52 and a female screw portion is formed to the fitted portion 53b at the distal end portion of the hard cylindrical portion 54.

As shown in (C) of FIG. 3, the proximal end portion of the flexible sheath 42 is mounted on the expanding portion 52. The expanding portion 52 has an expanding portion main body 52a and a tubular member 52b. On the inner circumferential surface of a proximal end portion of the expanding portion main body 52a, the above-mentioned fitting portion 53a is formed. On the outer circumferential surface of a proximal end portion of the tubular member 52b, a male screw portion 52e is formed which is fastened to the fitting portion 53a of the female screw portion. That is, the proximal end portion of the tubular member 52b is fitted to the above-mentioned fitting portion 53a inside the expanding portion main body 52a.

A distal end portion of the tubular member 52b is formed to have a thinner wall to the proximal end portion. In this case, level differences are formed on the outer circumferential surface between the proximal end portion and the distal end portion of the tubular member 52b. The inside diameter of the tubular member 52b is formed to be constant.

On the other hand, in the inside of the expanding portion main body 52a, there formed are level differences 52c and 52d which make the inside diameter on the proximal end side larger and the inside diameter more on the distal end side than that is made smaller than that on the proximal end side. The level differences 52c and 52d are expanded from the distal end side towards the proximal end side.

The proximal end portion of the sheath 42 is flared, and the inside diameter and the outside diameter thereof are expanded to be greater than the distal end portion of the sheath 42. The flared proximal end portion of the sheath 42 is arranged between level differences 52c and 52d of the expanding portion main body 52a and hooked to the level differences 52c and 52d.

Now, as described above, the male screw portion 52e of the proximal end portion of the tubular member 52b is fitted to the fitting portion 53a inside the expanding portion main body 52a. Consequently, the proximal end portion of the flared sheath 42 is arranged and pinched between the outer circumferential surface of the distal end portion of the tubular member 52b and the inner circumferential surface of the expanding portion main body 52a and between level difference portions 52c and 52d. That is, the sheath 42 is fixed to the expanding portion 52. For example, there is a case in which the distal end of the sheath 42 is applied with force from the distal end portion side to the proximal end portion side. In such a case, the proximal end portion of the sheath 42 is pinched between the expanding portion main body 52a and the tubular member 52b, and is brought in contact with the level difference 52c. This prevents the sheath 42 from being moved to the side of the proximal end portion of the dilator 40.

The inside diameter of the sheath 42 is formed in such a manner as to slidably adhere to the outer circumferential surface of the probe 24. The distal end portion of the sheath 42 has only the tip end 24a of the probe 24 defined to the protruded length when the handpiece unit 20 is mounted on the dilator 40. This makes it possible to prevent the side of the probe 24 that generates heat by vibration from coming into contact with, for example, a somatic wall when ultrasonic vibration is applied to the probe 24. That is, by the sheath 42 being arranged, heat of the probe 24 is prevented from being transmitted to the somatic wall.

Herein, explanation has been made on fixing the sheath 42 and the expanding portion 52 by the screw portion between the expanding portion main body 52a and the tubular member 52b. However, in the case where the sheath 42 and the expanding portion 52 can be firmly fixed, it may be bonded by use of, for example, adhesives. It is desirable to roughen the outer surface of the proximal end portion of the sheath 42 for easy bonding when bonding is carried out by use of adhesives in the case where the sheath 42 is formed with a member with high slidability, such as PTFE.

In addition, the sheath 42 is fixed to the proximal end portion of the expanding portion main body 52a, and is arranged along the inner circumferential surface of the distal end portion from the proximal end portion of the expanding portion main body 52a. That is, the sheath 42 has a long distance supported by the expanding portion 52. Consequently, the expanding portion main body 52a exhibits the function to stop breakage of the sheath 42.

The distal end portion of the expanding portion main body 52a is formed substantially in a taper-shape such that it expands smoothly to the outer circumferential surface of the sheath 42. In the dilator 40, the distal end portion of the expanding portion main body 52a is equipped with a short taper portion 56a in a shorter axial direction, a short cylindrical portion 56b formed integrally with a proximal end portion of the short taper portion 56a, and a long taper portion 56c formed integrally with a proximal end portion of the short cylindrical portion 56b.

The short taper portion 56a has the diameter expanded such that it coincides with the outside diameter of a distal end of the short cylindrical portion 56b from the distal end portion towards the proximal end portion. In this case, a distal end of the short taper portion 56a is smoothly expanded towards the proximal end portion side substantially free from level differences with respect to the outer circumferential surface of the sheath 42. Consequently, when a puncture hole is expanded by the dilator 40 after the puncture hole is formed by the probe 24, the distal end portion of the expanding portion main body 52a is prevented from getting stuck with the puncture hole.

The long taper portion 56c has the axial length longer than the short taper portion 56a, and the diameter thereof is smoothly expanded from the outside diameter of the short cylindrical portion 56b. The outside diameter of a proximal end of the long taper portion 56c coincides with the maximum outside diameter of the expanding portion main body 52a.

Herein, explanation has been made to providing the short cylindrical portion 56b, but the distal end portion of the expanded portion main body 52a may be configured to have one taper portion only, or as shown in (D) of FIG. 3, the taper portions 56a and 56c may be configured to be directly connected to each other.

Next, the cannula 70 will be explained.

The cannula 70 shown in (B) of FIG. 1 concentrically has a tubular insertion portion 72 to which the dilator 40 is inserted and a holding portion 74 provided to the proximal end portion (top end portion) of the insertion portion 72, the holding portion 74 being held by an operator. The insertion portion 72 of the cannula 70 is formed to be hard by a metal material such as, for example, stainless steel. The insertion portion 72 is formed into a thickness that can sufficiently stand the pressure inwards in the axial direction by the somatic wall.

As shown in (A) of FIG. 3 and (B) of FIG. 3 which shows the enlarged portion shown by arrow mark 3B in (A) of FIG. 3, the holding portion 74 has an engaging portion 76 on the inner circumferential surface thereof. The engaging portion 76 has a concave portion 76a annularly indented outwards into the axial direction on the inner circumferential surface of the holding portion 74. The protrusion 58 of the second attaching-and-detaching portion 50b of the dilator 40 is engaged to the concave portion 76a of the engaging portion 76.

On the top end portion of the holding portion 74, a check valve is mounted which prevents gases from the distal end portion side of the insertion portion 72 from passing through the holding portion 74. For example, a slit valve is used as the check valve. This makes it possible to keep the air pressure inside of, for example, a body cavity higher than that outside of a body.

As shown in (B) of FIG. 1, the distal end of the insertion portion 72 of the cannula 70 is cut obliquely such that it can be easily introduced into the somatic wall.

The inside diameter of the insertion portion 72 of the cannula 70 is formed slightly larger than the maximum outside diameter portion of the expanding portion 52 such that it can slide on the outer circumferential surface of the maximum outside diameter portion of the expanding portion 52 of the dilator 40. In addition, when the engaging portion 76 of the holding portion 74 of the cannula 70 is engaged with the protrusion 58 of the second attaching-and-detaching portion 50b of the dilator 40, the position of the distal end of the insertion portion 72 of the cannula 70 coincides with the outer circumferential surface of the expanding portion 52 of the dilator 40.

Next, explanation will be made on the assembly work for assembling the ultrasonic trocar 10 having such a configuration.

The tip end portion 24a of the probe 24 of the handpiece unit 20 is allowed to pass through the slit valve from the holding portion 48 of the proximal end portion of the dilator 40, and is inserted into the insertion portion 46. The first attaching-and-detaching portion 50a of the dilator 40 is fitted into a concave portion (not shown) of the lock mechanism portion 28 of the transducer 22. By depressing the engaging pin 28a, the first attaching-and-detaching portion 50a is arranged to the concave portion. Releasing the pressure against the engaging pin 28a under this condition engages the first attaching-and-detaching portion 50a. In this case, the inner circumferential surface of the sheath 42 of the dilator 40 is brought into close contact with the outer circumferential surface of the probe 24. The tip end 24a of the probe 24 is in a state of protruding from the distal end of the sheath 42 of the dilator 40. In this way, the handpiece unit 20 is engaged with the dilator 40.

In this manner, the distal end portion of a unit combining the handpiece unit 20 with the dilator 40 is allowed to pass through the check valve (slit valve) of the proximal end portion of the holding portion 74 of the cannula 70, and is inserted into the insertion portion 72. When the second attaching-and-detaching portion 50b of the holding portion 48 of the dilator 40 is inserted into a hole at the center of the check valve of the holding portion 74 of the cannula 70, the protrusion 58 of the second attaching-and-detaching portion 50b is engaged with the concave portion 76a of the engaging portion 76. In this way, the dilator 40 is engaged with the cannula 70.

In such a case, the top end surface of the holding portion 74 of the proximal end portion of the cannula 70 is in contact with the bottom surface of the flange portion 50 of the holding portion 48 of the dilator 40. The inner circumferential surface of the distal end portion of the insertion portion 72 of the cannula 70 is in a state substantially in close contact with the outer circumferential surface of the maximum outside diameter portion of the expanding portion 52 of the dilator 40.

Then, the handpiece unit 20, dilator 40, and cannula 70 are integrally assembled to form the ultrasonic trocar 10 shown in (A) of FIG. 1. To the connector portion 26 of the handpiece unit 20 of the ultrasonic trocar 10, the power supply unit 32 and the switch 34 are connected.

It has been explained that a member having the handpiece unit 20 engaged with the dilator 40 is inserted through the cannula 70, but it is also preferred that the handpiece unit 20 is inserted through a member having the dilator 40 inserted through the cannula 70.

Next, a method (function) of operating the ultrasonic trocar 10 will be explained.

The switch 34 which is input means for actuating an output control mechanism of the power supply unit 32 is operated. Then, electrical energy is supplied from the power supply unit 32 to a piezoelectric element of the transducer 22. The piezoelectric element generates mechanical vibration in accordance with the energy amount. This vibration is amplified by the horn inside the transducer 22 and the probe 24 arranged on the distal end portion side of the transducer 22, and the maximum amplitude is output by the tip end 24a of the probe 24.

In this state, the tip end 24a of the probe 24 is inserted from the somatic wall to the body inside. That is, first, the distal end portions of the probe 24 and the sheath 42 are punctured into the somatic wall to form a small diameter puncture hole. In this manner, the tip end 24a of the probe 24 of the ultrasonic trocar 10 is inserted from the somatic wall to the body inside. Under this condition, the switch 34 is operated, and the supply of electrical energy to the transducer 22 is stopped. That is, the supply of ultrasonic vibration to the probe 24 is stopped.

The ultrasonic trocar 10 is inserted further into the somatic wall and is inserted until the distal end of the short taper portion 56a at the distal end of the expanding portion 52 of the dilator 40 comes in contact with the puncture hole. Because in this case, the proximal end portion of the sheath 42 is pinched between the expanding portion main body 52a and the tubular member 52b, the sheath 42 is arranged in a body cavity in such a manner that the sheath 42 is prevented from moving to the proximal end portion side.

In this state, the engaging pin 28a of the lock mechanism portion 28 of the handpiece unit 20 is depressed. That is, engagement with the first attaching-and-detaching portion 50a of the dilator 40 in the concave portion of the distal end portion of the transducer 22 of the handpiece unit 20 is released. The probe 24 of the handpiece unit 20 is pulled out from the lumens of the dilator 40 and the cannula 70.

Thereafter, dilation is carried out by the expanding portion 52 of the dilator 40. Herein, by means of the expanding portion 52 of the dilator 40, a hole diameter of the puncture hole formed by the probe 24 is expanded substantially to the outside diameter of the insertion portion 72 of the cannula 70. That is, the expanding portion 52 is introduced into the somatic wall by press-fitting.

At this time, the short taper portion 56a of the distal end of the expanding portion 52 has the diameter smoothly expanded with respect to the sheath 42. Consequently, when the expanding portion 52 is pressure-fitted to expand the puncture hole by the expanding portion 52, it is possible for the maximum outside diameter position of the dilator 40, that is, the distal end portion of the insertion portion 72 of the cannula 70 to easily puncture the somatic wall. In this case, since the short taper portion 56a is easily pressure-fitted to the somatic wall, the short cylindrical portion 56b and the long taper portion 56c can easily puncture the somatic wall. For this reason, a puncture can be easily made in the somatic wall to the maximum outside diameter of the expanding portion 52 of the dilator 40. Consequently, the distal end portion of the insertion portion 72 of the cannula 70 closely in contact with the outside of the maximum outside diameter portion of the expanding portion 52 can easily make a puncture to the somatic wall.

After the insertion portion 72 of the cannula 70 is introduced into the somatic wall, the operator releases engagement between the dilator 40 and the cannula 70 while holding the holding portion 74 so as to prevent the cannula 70 from moving. In such a case, the engaging portion 76 of the cannula 70 is the concave portion 76a, and the second attaching-and-detaching portion 50b of the dilator 40 is the protrusion 58 (convex portion). In particular, the body portion 44 of the dilator 40 is preferably formed with a resin material which can be easily elastically deformed. For this reason, the engagement between the cannula 70 and the dilator 40 is easily released. Needless to say, even in the case where the body portion 44 is formed with a metal material, engagement between the cannula 70 and the dilator 40 can be easily released.

At this time, the dilator 40 is removed from a through-hole of the cannula 70. Thus, only the cannula 70 is retained to the somatic wall.

Under this condition, an endoscope, a treatment device and the like are inserted into the cannula 70, and various treatments are carried out. In this case, the pressure inside the body cavity is maintained to a predetermined pressure by the check valve (slit valve) provided to the holding portion 74 of the cannula 70. After the completion of treatment, the cannula 70 is removed.

The handpiece unit 20 and the cannula 70 are disassembled to the disassembly range, respectively, and washed and disinfected. For example, in the handpiece unit 20, the probe 24 is removed from the ultrasonic transducer 22. The check valve is removed from the cannula 70. In this way, the handpiece unit 20 and the cannula 70 are washed and disinfected, and then reused.

On the other hand, in the dilator 40, the insertion portion 46 is separated into the expanding portion 52 and the cylindrical portion 54 by allowing the body portion 44 to relax the fastened state of the screw. In addition, the sheath 42 is detached from the expanding portion 52. At this time, the state of the sheath 42 mounted on the expanding portion 52 is confirmed. In the case where the distal end of the sheath 42 is, for example, in a rolled-up state or in another unreusable state, the sheath 42 is discarded. In the case where the sheath 42 is judged to be reusable, on the other hand, the sheath 42 is washed and disinfected as well.

In the case where the sheath 42 is judged unreusable after washing and disinfection, the sheath 42 is discarded.

The washed and disinfected sheath 42 or a newly prepared sheath 42 is mounted to the expanding portion 52. The expanding portion 52 in this state is fastened to the cylindrical portion 54 of the insertion portion 46 again, and the dilator 40 is formed again.

Incidentally, in the case where a cannula 70 of different kind is used, that is, in the case where a cannula 70 whose inside diameter of the insertion portion 72 thereof is different is used, the maximum outside diameter of the expanding portion 52 of the dilator 40 may be also changed to the inside diameter of the insertion portion 72 of the cannula 70. This can be achieved by forming and shaping, for example, the female screw portion 53a of the inner circumferential surface of the proximal end portion of the expanding portion 52 of the dilator 40 so as to always coincide with, for example, the male screw portion 53b of the outer circumferential surface of the distal end portion of the insertion portion 46. That is, this can be achieved when the proximal end portion side from the cylindrical portion 54 of the body portion 44 of the dilator 40 is formed with common parts.

In this way, the handpiece unit 20, dilator 40, and cannula 70 of the ultrasonic trocar 10 are washed and disinfected, or have part of components replaced to reconstruct component parts 20, 40, and 70 of the ultrasonic trocar 10.

As described above, according to this embodiment, the following effects can be obtained.

Because the sheath 42 is integrated into the dilator 40 which expands the puncture hole to the diameter of the insertion portion 72 of the cannula 70, the ultrasonic trocar 10 can be configured by assembling only three components 20, 40 and 70. For this reason, the assembling work and removing work of the ultrasonic trocar 10 can be easily carried out. This can improve the maneuverability of the ultrasonic trocar 10, and at the same time, can achieve light weight and reduce the cost. In particular, in the case where the body portion 44 of the dilator 40 is formed with a resin material, light weight can be achieved.

Since the short taper portion 56a and the short cylindrical portion 56b are arranged to the distal end of the long taper portion 56c of the distal end portion of the expanding portion 52, it is possible to suppress level differences with the outer circumferential surface of the sheath 42 of the distal end of the expanding portion 52, so that the insertability of the expanding portion 52 can be improved when the puncture hole of the somatic wall is expanded. More specifically, since the outside diameter of the distal end portion of the expanding portion 52 is smoothly expanded with respect to the outside diameter of the sheath 42 from the distal end to the proximal end, it is possible to easily cause the distal end of the expanding portion 52 to puncture a somatic wall after the sheath 42 punctures the somatic wall. Therefore, the puncture hole can be further expanded easily by use of the short cylindrical portion 56b and the long taper portion 56c. Note that the same holds for the case in which no short cylindrical portion 56b is provided and the short taper portion 56a and the long taper portion 56c are directly connected.

In addition, the expanding portion 52 is made attachable to and detachable from the cylindrical portion 54 of the insertion portion 46. Consequently, it is possible to use the expanding portion 52 in conformity to the inside diameter of the cannula 70. That is, an expanding portion 52 with varying diameters can be mounted on the distal end of the cylindrical portion 54.

Further, because the expanding portion 52 is made attachable to and detachable from the cylindrical portion 54 of the insertion portion 46 while the sheath 42 is made attachable to and detachable from the expanding portion 52, it is possible to replace a material with low rigidity, such as sheath 42 as required. Herein, description has been made on the case in which only the sheath 42 is replaced when the sheath 42 is not reused, but for example, the expanding portion 52 may be discarded together with the sheath 42. In addition, in the case where the dilator 40 is formed with a resin material, the dilator 40 which has been used may be discarded as a disposable dilator.

Now, a second embodiment will be described with reference to FIG. 4. This embodiment is a modified example of the first embodiment, and the same functional components as those in the first embodiment are denoted by the same reference number, and detailed description thereof is omitted.

An ultrasonic trocar 10 according to this embodiment is different from that of the first embodiment in the configuration of the dilator 40.

As shown in (A) to (C) of FIG. 4, the dilator 40 has a body portion 44a and a sheath 42a. A removable holding portion 48a is arranged to a proximal end portion of the body portion 44a.

The holding portion 48a of the dilator 40 has a first attaching-and-detaching portion 50a on the top side of a flange portion 50. As shown in (B) of FIG. 4, a body portion mounting portion 62 which removably fixes the proximal end portion of the body portion 44a is formed below the flange portion 50 of the holding portion 48a. As shown in (C) of FIG. 4, still below the body portion mounting portion 62, a sheath mounting portion 64 which removably fixes the proximal end portion of the sheath 42a is formed. That is, the sheath 42a is formed to be longer than the body portion 44a such that it is inserted through the body portion 44a.

The proximal end portion of the sheath 42a is flared and mounted to the sheath mounting portion 64. In such a case, the sheath mounting portion 64 and the proximal end portion of the sheath 42a are, for example, tightened by screws and fixed by a click mechanism or adhesive bonding.

On the external circumference of the sheath 42a, the body portion 44a is arranged. The body portion 44a is integrally formed by, for example, a resin material. The proximal end portion of the body portion 44a is, for example, flared as is the case of the proximal end portion of the sheath 42a. The body portion mounting portion 62 of the holding portion 48a and the proximal end portion of the body portion 44a are, for example, tightened by screws and fixed by a click mechanism or adhesive bonding.

Note that the distal end portion of the expanding portion 52 of the body portion 44a is equipped with, for example, a short taper portion 56a, a short cylindrical portion 56b and a long taper portion 56c. In addition, it is desirable to cover and reinforce the inner circumferential surface of these short taper portion 56a, short cylindrical portion 56b, and long taper portion 56c as well as the outer circumferential surface of the sheath 42 by reinforcement members (not shown) of a tubular rubber material, a resin material or the like. Thus, the reinforcement members serve as breakage stoppers of the sheath 42 together with the expanding portion 52. In this way, the inner circumferential surface of the distal end of the body portion 44a is in close contact with the external circumferential surface of the sheath 42a.

As shown in (D) of FIG. 4, a concave portion 66 is formed annularly on the external circumferential surface of the body portion 44a of the dilator 40. A C-ring 68 whose diameter can be expanded and contracted is fitted to the concave portion 66. The C-ring 68 expands from the contracted state when the dilator 40 is engaged with the concave portion 76a of the engaging portion 76 on the inner circumferential surface of the cannula 70, so that the dilator 40 and the cannula 70 are engaged with each other.

In the case where engagement between the dilator 40 and the cannula 70 is released, a press button (not shown) provided on the outside of the cannula 70 is depressed, and the dilator 40 may be pulled out with the C-ring 68 contracted.

As described above, according to this embodiment, the following effects can be obtained.

It is possible to use the dilator 40 in the same manner as in the first embodiment with the proximal end portion of the sheath 42a and the body portion 44a fixed by the holding portion 48a.

In the case where the body portion 44a of the dilator 40 is formed with a metal material, the dilator can be washed, disinfected and reused. On the other hand, in the case where the body portion 44a is made of a resin material, the dilator may be washed, disinfected and reused or may be arranged as it is as a disposable dilator as well.

Note that the expanding portion 52 and the cylindrical portion 54 of the body portion 44a of the dilator 40 may be made attachable to and detachable from each other. In this case, the expanding portion 52 may be selected and used in accordance with the inside diameter of the insertion portion 72 of the cannula 70.

Now, a third embodiment will be explained with reference to FIG. 5. This embodiment is a modified example of the second embodiment, and the same functional components as those in the first embodiment are denoted by the same reference number, and detailed description thereof is omitted.

As shown in (A) of FIG. 5, a body portion 44b of a dilator 40 has an insertion portion 46b and a holding portion 48b integrally formed. The body portion 44b is formed by molding by use of a resin material such as, for example, polyphenyl sulfone.

At a proximal end portion of the insertion portion 46b, an engaging portion 69 having a pawl 69a that protrudes outwards in the radial direction shown in (B) of FIG. 5 is integrally formed with the body portion 44b. It is preferable to provide not only one engaging portion but also a plurality of engaging portions 69 as shown in (C) of FIG. 5.

In this case, as shown in (B) of FIG. 3, the pawl 69a of the engaging portion 69 of the body 44b of the dilator 40 is engaged with the concave portion 76a of the engaging portion of 76 of the holding portion 74 of the cannula 70.

Although not shown, the sheath 42 has the body portion 44b of the dilator 40 inserted therethrough, and is fixed to the holding portion 48 of the proximal end portion of the body portion 44b.

As described above, according to this embodiment, the following effects can be obtained.

Because the body portion 44b of the dilator 40 is integrally formed, the cost required for fabricating the dilator 40 can be reduced. Consequently, the dilator 40 which has been used can be treated to be disposable.

Further, because pawls 69b can be integrally molded to the body portion 44b simultaneously, the cost can be reduced still more. For this reason, the dilator 40 may be preferably made disposable.

Several embodiments have been described in detail, with reference to the drawings. Nevertheless, the present invention is not limited to the embodiments described above. The present invention includes any embodiments that can be achieved without departing the scope and spirit of the invention.

### Industrial Applicability

The present invention can provide an ultrasonic trocar to be able to be easily carried out the assembling work and removing work.

## Claims

1. An ultrasonic trocar (10) **characterized by** comprising:
a handpiece unit (20) having an ultrasonic transducer (22) and a probe (24) which has a tip end and a proximal end, the transducer being connected to the proximal end, the handpiece unit generating ultrasonic vibration by the transducer, and with ultrasonic vibration transmitted from the proximal end to the tip end of the probe, puncturing the tip end of the probe into a somatic wall to form a puncture hole in the somatic wall;
a dilator (40) including a tubular body portion (44) and a sheath (42), the body portion having distal and proximal end portions and an expanding portion (52) which expands the puncture hole, the probe is inserted from the proximal end portion of the body portion, and the sheath is arranged in a state of protruding from the distal end portion of the body portion and covers a peripheral surface of the probe with the tip end of the probe left.
a tubular cannula (70) through which the dilator is inserted and kept retained in the puncture hole expanded by the expanding portion.

2. The ultrasonic trocar (10) according to claim 1, **characterized in that** the expanding portion (52) includes a taper portion (56a, 56b, 56c) which smoothly expand from the distal end portion to the proximal end portion of the body portion (44) with respect to the peripheral surface of the sheath (42).

3. The ultrasonic trocar (10) according to claim 1 or 2, **characterized in that** the body portion (44) includes:
an insertion portion (46) having the distal end portion and the expanding portion (52); and
a holding portion (48) which has the proximal end portion and has an engaging portion (50a, 50b) to which the handpiece unit (20) and the cannula (70) are removably engaged.

4. The ultrasonic trocar (10) according to claim 1 or 2, **characterized in that** the body portion (44) integrally includes an engaging portion (50b) to which the cannula (70) is removably engaged.

5. The ultrasonic trocar (10) according to claim 3 or 4, **characterized in that** the insertion portion (46) further includes a cylindrical portion (54) between the expanding portion (52) and the holding portion (48), and
the cylindrical portion and the expanding portion are attachable to and detachable from each other.

6. The ultrasonic trocar (10) according to any one of claims 1 to 5, **characterized in that** the expanding portion (52) includes sheath a holding portion (53a, 53b) which hold the sheath (42).

7. The ultrasonic trocar (10) according to any one of claims 1 to 5, **characterized in that** the body portion (44) and the sheath (42) includes loading and unloading mechanism (53a, 53b; 64) which are attachable to and detachable from each other.

8. The ultrasonic trocar (10) according to any one of claims 1 to 4, **characterized in that** the body portion (44) is integrally formed by a resin material.

9. A method for using an ultrasonic trocar (10) which retains a cannula (70) of an ultrasonic trocar to a somatic wall, the method comprising:
attaching a probe (24) to an ultrasonic transducer (22) and forming a handpiece unit;
inserting the probe of the handpiece unit (20) through a dilator (40) removably arranged on the outside of the handpiece unit, the dilator having a body portion (44) equipped with an expanding portion (52), and a sheath (42) which protrudes from a distal end of the body portion;
inserting the dilator, through which the handpiece unit has been inserted, through the cannula (70) retained to the somatic wall;
generating ultrasonic vibration by the transducer, and transmitting the ultrasonic vibration from a proximal end to a tip end of the probe connected to the transducer;
pressing the tip end of the probe against the somatic wall and forming a puncture hole in the somatic wall;
removing the handpiece unit from the dilator;
arranging the cannula to the puncture hole while the puncture hole is gradually expanded by the expanding portion of the dilator; and
removing the dilator from the cannula.

10. A method for using an ultrasonic trocar (10) which retains a cannula (70) of an ultrasonic trocar to a somatic wall, the method comprising:
inserting a dilator (40) through the cannula (70) retained to the somatic wall, the dilator having a body portion (44) equipped with an expanding portion (52), and a sheath (42) which protrudes from a distal end of the body portion;
attaching a probe (24) to an ultrasonic transducer (22) and forming a handpiece unit;
inserting the probe of the handpiece unit (20) through the dilator;
generating ultrasonic vibration by the transducer, and transmitting the ultrasonic vibration from a proximal end to a tip end of the probe connected to the transducer;
pressing the tip end of the probe against the somatic wall and forming a puncture hole in the somatic wall;
removing the handpiece unit from the dilator;
arranging the cannula to the puncture hole while expanding the puncture hole by the expanding portion of the dilator; and
removing the dilator from the cannula.
